# DEMANDE DE BREVET EUROPEEN

(11) **EP 1 532 944 A1**
(43) Date de publication de la demande: **25.05.2005**
(21) Numéro de dépôt: 04356171.1
(22) Date de dépôt: 22.10.2004
(51) Int. Cl.: A61F 2/16

(54) **Implant oculaire pour la correction des défauts de la vision**

(30) Priorité: 21.11.2003 FR 0313668
(71) Demandeur: Duvert, Jacques, 69005 Lyon (FR)
(72) Inventeur: Duvert, Jacques, 69005 Lyon (FR)
(74) Mandataire: Bratel, Gérard

(57) **Abrégé**

Cet implant oculaire permet la correction de diverses amétropies. Il comprend, sur ou dans un même support (6) implantable dans un oeil (4), une première lentille (9) fixe, définissant un axe optique (A), et une seconde lentille (11) mobile, déplaçable entre une position écartée latéralement de la première lentille (9) et une position dans laquelle elle se superpose, suivant le même axe optique (A), à la première lentille (9). Une position est notamment prévue pour la vision de loin, et l'autre position pour la vision de près. Le déplacement de la seconde lentille (11), entre ses deux positions, peut être commandé depuis l'extérieur par des moyens magnétiques.

## Description

La présente invention concerne, de manière générale, les implants oculaires qui sont posés pour corriger divers défauts habituels de la vision, aussi désignés comme amétropies, tels que : astigmatisme, hypermétropie, myopie, presbytie, post aphakie.

Le mode de correction traditionnel des défauts de la vision consiste dans le port de lunettes correctrices. Certains types de défaut nécessitent des verres de lunettes bifocaux ou progressifs, qui peuvent occasionner une gêne pour leur utilisateur. D'une façon générale, les lunettes ont pour inconvénients de se rayer, de nécessiter de fréquents nettoyages, de se casser et de pouvoir être perdues. De plus, dans le cas de verres bifocaux ou progressifs, destinés à la correction de la presbytie, la lecture en position allongée, par exemple au lit, s'avère difficile car la vision de près est assurée par la partie basse de tels verres de lunettes.

Les lunettes traditionnelles sont, de plus en plus, remplacées par des lentilles de contact, ou lentilles "cornéennes", qui ont bénéficié récemment de divers progrès et évolutions : lentilles souples, lentilles jetables, lentilles progressives ou multifocales. Toutefois, les lentilles de contact sont mal tolérées par certaines personnes, l'utilisation de lentilles progressives ou multifocales nécessite un temps d'accoutumance (apprentissage cérébral) assez long, parfois ces lentilles ne donnent pas une vision parfaite de près, et enfin les lentilles ne dispensent pas toujours du port occasionnel de lunettes.

Une autre voie de recherche récente a été le recours à diverses techniques opératoires, consistant à remodeler l'oeil, en particulier la cornée, pour corriger des défauts de la réfraction oculaire. Ces techniques opératoires sont, entre autres : le keratomileusis, l'épikeratophakie, la kératotomie radiaire, la chirurgie "LASIK" (Laser Assisted In-Situ Keratomileusis), et la pose d'implants notamment sous forme de lentille intra-cornéenne, qui reste encore à ce jour un domaine expérimental. De telles techniques opératoires restent toutefois délicates et aléatoires, et ne garantissent donc pas un résultat certain et parfait, même de manière différée après cicatrisation. Elles peuvent donner des complications comme l'effet "haze", ou fragiliser l'oeil, avec des conséquences irréversibles. Avec cette méthode, on peut opérer les deux yeux de manière inégale, en cas de presbytie, en laissant un ceil légèrement myope pour lui permettre de focaliser de près, et en donnant à l'autre oeil une vision de loin normale, mais là aussi il en résulte un inconfort visuel et une nécessité d'adaptation cérébrale. En ce qui concerne les implants, ceux-ci peuvent être posés à l'intérieur de l'oeil soit après ablation du cristallin, soit derrière la cornée sans enlever le cristallin. Ces implants sont généralement monofocaux, auquel cas ils restituent une excellente qualité de vision, mais l'autonomie en vision de près comme de loin passe nécessairement par la monovision. Il a aussi été proposé des implants oculaires multifocaux, qui permettent une vision de près et de loin, mais avec une qualité de vision légèrement moins bonne, et la nécessité d'un apprentissage cérébral. Enfin, les implants oculaires connus ne permettent pas la correction de l'astigmatisme.

La présente invention vise à remédier à l'ensemble des inconvénients précédemment exposés, en fournissant une solution nouvelle du genre implant oculaire, évitant tous les problèmes actuellement posés par les lunettes, les lentilles de contact, la chirurgie et les implants déjà connus, la solution proposée permettant la correction de toutes les amétropies y compris l'astigmatisme et, en particulier, permettant par elle-même la vision de loin comme la vision de près avec un champ de vision complet.

A cet effet, l'invention a pour objet un implant oculaire pour la correction des défauts de la vision, qui comprend essentiellement, sur ou dans un même support implantable dans un oeil, une première lentille ou optique équivalente fixe, définissant un axe optique, et une seconde lentille ou optique équivalente mobile, déplaçable entre une position écartée latéralement de la première lentille et une position dans laquelle elle se superpose, suivant le même axe optique, à la première lentille.

Ainsi, l'implant oculaire, objet de l'invention possède deux positions fonctionnelles, résultant de la disposition relative de ses deux lentilles, et correspondant respectivement à deux distances focales :
- dans une première position, pour laquelle la seconde lentille est écartée de l'axe de la première lentille, la vision se fera par la seule première lentille, ceci correspondant notamment à la vision de loin ;
- dans une deuxième position, pour laquelle la seconde lentille se superpose à la première lentille, la vision se fera au travers de la combinaison des deux lentilles, ceci correspondant notamment à la vision de près.

On notera que l'axe optique défini par la première lentille fixe coïncide avec l'axe pupillaire, lorsque l'implant oculaire est en place dans l'oeil.

Dans un mode de réalisation préféré de l'invention, la seconde lentille est montée coulissante dans des guides du genre rails ou glissières, formés sur le support précité, de manière à être déplaçable par translation entre sa position écartée de la première lentille et sa position superposée à la première lentille.

Avantageusement, le déplacement de la seconde lentille, entre sa position écartée de la première lentille et sa position superposée à la première lentille, est commandable à volonté depuis l'extérieur par des moyens magnétiques. Par exemple, la seconde lentille comporte un filament métallique incrusté, ou entourant cette lentille, tandis que le moyen de commande extérieur est constitué par un objet tel qu'une bague, porté ou tenu par l'utilisateur, et muni d'un aimant permanent créant un champ magnétique extérieur, agissant sur ledit filament. Ainsi, en déplaçant convenablement devant son oeil le doigt, muni de la bague appropriée, l'utilisateur pourra faire passer cet implant oculaire de la position "vision de loin" à la position "vision de près", et vice-versa.

Le support des deux lentilles est avantageusement constitué par une cage ou analogue, dont les orifices laissent passer à l'intérieur l'humeur aqueuse de l'oeil. La cage, ou autre support analogue, est munie d'au moins une haptique réalisable selon des principes connus, pour le maintien en place de l'implant oculaire.

Cet implant oculaire peut être fabriqué en divers matériaux, hydrophiles ou hydrophobes, de préférence suffisamment souples pour que l'implant soit pliable, ce qui facilitera en particulier sa pose. Ainsi, un implant souple pourra être posé en le faisant pénétrer par une incision de 3 millimètres au maximum en pleine cornée, car dans le cas contraire il y aurait un astigmatisme résiduel après opération. Toutefois, un implant souple pourrait se coincer, s'il n'est pas bien posé, et l'on pourra éviter ce risque en prévoyant une catégorie d'implants rigides ; dans ce cas, l'incision se fera dans le limbe cornéen, et pourra être supérieure à 3 millimètres, pour éviter tout astigmatisme résiduel après opération.

L'implant oculaire objet de l'invention peut être posé dans la chambre postérieure de l'oeil, en arrière de l'iris et en avant du cristallin, ceci notamment pour la correction de la presbytie (sans cataracte).

Il est à noter que, contrairement aux implants oculaires existants, l'implant de la présente invention permet de corriger aussi l'astigmatisme dans la vision de loin, donc de corriger dans ce cas toutes les amétropies ou défauts de la vision. Ceci est notamment obtenu avec une lentille cylindrique ou sphéro-cylindrique, qui est par exemple la lentille mobile.

En cas d'opération de la cataracte, l'implant oculaire objet de l'invention peut être placé dans la capsule cristallinienne.

Dans tous les cas, l'implant oculaire objet de l'invention permet, en vision de loin comme en vision de près, de disposer d'un champ de vision complet, contrairement aux verres de lunettes de type bifocal ou progressif, qui ne permettent de bien voir de près que si l'on regarde vers le bas.

L'invention sera mieux comprise à l'aide de la description qui suit, en référence au dessin schématique annexé représentant, à titre d'exemple, une forme d'exécution de cet implant oculaire pour la correction des défauts de la vision :
Figure 1 représente, en coupe très schématique, un implant oculaire conforme à la présente invention, posé dans un oeil ;
Figure 2 est une vue en perspective de cet implant oculaire ;
Figure 3 en est une de face, dans une première position d'utilisation ;
Figure 4 en est une vue par l'arrière, dans la même position ;
Figure 5 en est une vue en coupe, suivant V-V de figure 4 ;
Figure 6 est une vue en coupe similaire à la figure 5, mais illustrant une deuxième position d'utilisation de l'implant oculaire.

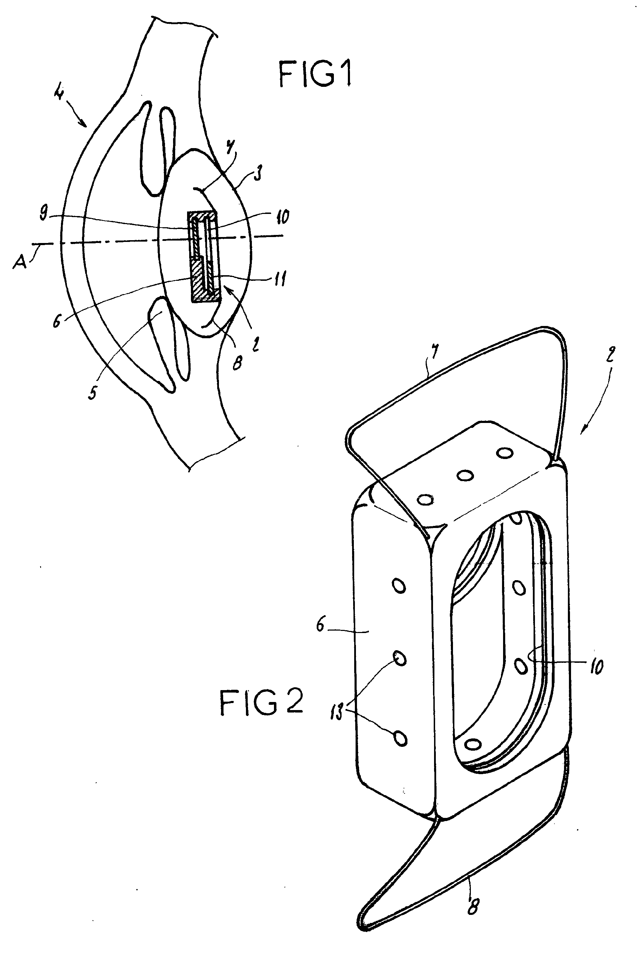

La figure 1 montre un implant oculaire, désigné globalement par la référence 2, qui est placé à l'intérieur de la capsule cristallinienne 3 d'un oeil humain 4, en arrière de l'iris 5.

En se référant aussi aux figures suivantes 2 à 6, l'implant oculaire 2 comporte un support évidé ou "cage" 6, de forme oblongue, muni extérieurement d'haptiques 7 et 8 pour son maintien en place.

Sur le support 6 est montée, en position fixe, une première lentille optique 9, qui définit un axe optique A. Lorsque l'implant oculaire 2 est en place, cet axe A coïncide avec l'axe pupillaire.

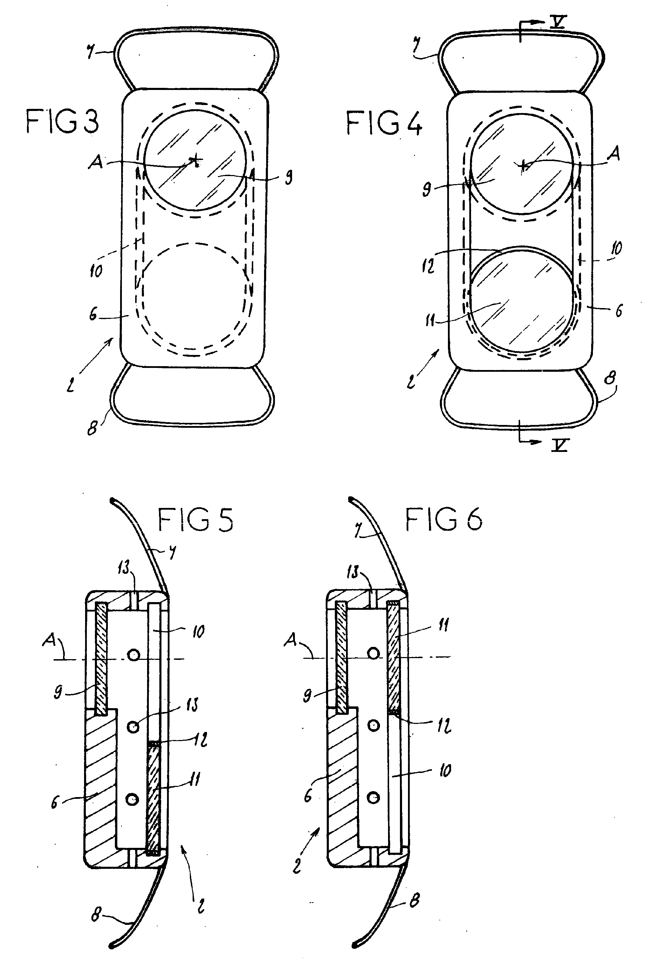

Le support 6 forme, intérieurement, deux glissières latérales parallèles 10, dans lesquelles est montée coulissante une seconde lentille optique 11, laquelle a été omise sur la figure 2. La seconde lentille 11 est ainsi déplaçable, dans un plan parallèle à celui de la première lentille 9, entre une position écartée latéralement de la première lentille 9 (figures 3 à 5), et une autre position dans laquelle elle se superpose à la première lentille 9 (figure 6).

La seconde lentille 11 est pourvue d'un filament métallique circulaire 12, incrusté dans ladite lentille ou entourant cette lentille 11, qui permet de la déplacer en translation alternativement, d'une position à l'autre et vice-versa, à l'aide d'un champ magnétique extérieur, par exemple produit par une bague munie d'un aimant permanent et portée au doigt par l'utilisateur. Ainsi, en passant son doigt convenablement devant l'oeil 4, l'utilisateur peut "commuter" l'implant oculaire 2 d'une position à l'autre.

Dans la première position (figures 3 à 5), seule la première lentille 9 est présente sur l'axe pupillaire (axe optique A), l'autre lentille 11 en étant écartée latéralement. La première lentille 9 assure alors, à elle seule, la correction appropriée pour la vision de loin.

Dans l'autre position (figure 6), la seconde lentille 11 se superpose à la première lentille 9, suivant le même axe optique A, et l'ensemble des deux lentilles 9 et 11 assure alors la correction appropriée pour la vision de près, la seconde lentille 12 étant dimensionnée en conséquence.

Bien entendu, les deux lentilles 9 et 11 sont de type adapté (lentilles convergentes ou divergentes) et de distance focale appropriée à chaque cas, leur représentation comme lentilles cylindriques avec une épaisseur constante étant ici purement symbolique. Par exemple, la presbytie est corrigée d'emblée à + 3,5 dioptries par la seconde lentille 11.

L'implant oculaire 2, précédemment décrit, est réalisable dans des matériaux tels que silicone, polyméthacrylate (PMMA), copolymère de diméthylamide, polyvinyle..., le matériau pouvant être hydrophile ou hydrophobe, et plus ou moins souple. L'utilisation d'une "cage" ajourée, ou d'un support 6 pourvu d'orifices 13, permet de laisser passer l'humeur aqueuse de l'oeil 4.

L'épaisseur de l'implant oculaire 2, objet de l'invention, sera de 3 millimètres au maximum, en particulier pour un implant destiné à la correction de la presbytie car, dans ce cas, l'implant sera posé entre le cristallin et l'iris, où il disposera de peu de place.

On ne s'éloignerait pas du cadre de l'invention, telle que définie dans les revendications annexées :
■ en donnant à l'implant oculaire, notamment à son support et à ses haptiques, toutes formes de détail ;
■ en réalisant cet implant oculaire en tous matériaux appropriés, ne présentant pas d'intolérances ;
■ en inversant les rôles des deux lentilles c'est-à-dire en prévoyant la lentille fixe pour la vision de près, et la lentille mobile (amenée en coïncidence avec la lentille fixe) pour la vision de loin ;
■ en destinant cet implant oculaire à la correction de tous types d'amétropies : hypermétropie, myopie, presbytie, aphakie, combinées ou non à l'astigmatisme, ce dernier étant corrigé en ajustant les lentilles de l'implant dans l'axe correspondant, de la même manière que l'opticien ajuste des verres de lunette sur une monture ;
■ en posant cet implant dans l'oeil, en toute situation convenable, par exemple entre l'iris et le cristallin, le mode d'implantation étant fonction de la position retenue et pouvant appliquer toutes techniques opératoires, de préférence de manière à permettre un remplacement ultérieur de l'implant oculaire.

## Revendications

1. Implant oculaire pour la correction des défauts de la vision, ou amétropies, **caractérisé en ce qu'**il comprend, sur ou dans un même support (6) implantable dans un oeil, une première lentille (9) ou optique équivalente fixe, définissant un axe optique (A), et une seconde lentille (11) ou optique équivalente mobile, déplaçable entre une position écartée latéralement de la première lentille (9) et une position dans laquelle elle se superpose, suivant le même axe optique (A), à la première lentille (9), le déplacement de la seconde lentille (11) entre les deux positions étant commandable depuis l'extérieur.

2. Implant oculaire selon la revendication 1, **caractérisé en ce que** la seconde lentille (11) est montée coulissante dans des guides du genre rails ou glissières (10), formés sur le support (6), de manière à être déplaçable par translation entre sa position écartée de la première lentille (9), et sa position superposée à la première lentille (9).

3. Implant oculaire selon la revendication 1 ou 2, **caractérisé en ce que** le déplacement de la seconde lentille (11), entre sa position écartée de la première lentille (9) et sa position superposée à la première lentille (9), est commandable depuis l'extérieur par des moyens magnétiques.

4. Implant oculaire selon la revendication 3, **caractérisé en ce que** la seconde lentille (11) comporte un filament métallique (12) incrusté, ou entourant cette lentille (11), tandis que le moyen de commande extérieur est constitué par un objet tel qu'une bague porté ou tenu par l'utilisateur, et muni d'un aimant permanent créant un champ magnétique extérieur agissant sur ledit filament (12).

5. Implant oculaire selon l'une quelconque des revendications 1 à 4, **caractérisé en ce que** le support (6) des deux lentilles (9, 11) est constitué par une cage ou analogue, dont les orifices (13) laissent passer à l'intérieur l'humeur aqueuse.

6. Implant oculaire selon l'une quelconque des revendications 1 à 5, **caractérisé en ce qu'**il est fabriqué en un matériau souple, rendant cet implant oculaire (2) "pliable" en particulier pour sa pose.

7. Implant oculaire selon l'une quelconque des revendications 1 à 6, **caractérisé en ce que** la première position, pour laquelle la seconde lentille (11) est écartée de la première lentille (9), est prévue pour la vision de loin, se faisant alors par la seule première lentille (9), tandis que la seconde position, pour laquelle la seconde lentille (11) se superpose à la première lentille (9), est prévue pour la vision de près, la vision se faisant alors au travers de la combinaison des deux lentilles (9, 11).
